# EUROPÄISCHE PATENTSCHRIFT

(11) **EP 3 119 270 B1**
(45) Veröffentlichungstag und Bekanntmachung des Hinweises auf die Patenterteilung: **12.02.2020**
(21) Anmeldenummer: 15716394.0
(22) Anmeldetag: 13.03.2015
(51) Int. Cl.: A61B 5/00

(54) **VORRICHTUNG FÜR DIE ERMITTLUNG DES ZUSTANDES DER HAUT EINER PERSON**
DEVICE FOR DETERMINING THE STATE OF A PERSON'S SKIN
DISPOSITIF POUR DÉTERMINER L'ÉTAT DE LA PEAU D'UNE PERSONNE

(30) Priorität: 17.03.2014 AT 501912014
(43) Veröffentlichungstag der Anmeldung: 25.01.2017
(73) Patentinhaber: AIT Austrian Institute of Technology GmbH, 1220 Wien (AT)
(72) Erfinder: OBERLEITNER, Andreas, 2492 Zillingdorf (AT); LURF, Robert, 2640 Gloggnitz (AT); MEINDL, Michael, 2632 Wimpassing (AT); BEISTEINER, Martin, 2853 Bad Schönau (AT)
(74) Vertreter: Wildhack & Jellinek
(86) Internationale Anmeldenummer: PCT/AT2015/050064
(87) Internationale Veröffentlichungsnummer: WO 2015/139065

(56) Entgegenhaltungen:
- NL-C- 2 009 123
- US-A1- 2006 052 678
- US-A1- 2011 178 375

## Beschreibung

Gegenstand der vorliegenden Erfindung ist eine Vorrichtung für die Ermittlung des Zustandes der Haut einer Person mit einem an zumindest einer Stelle der Haut auf dieselbe unter Bindung an dieselbe aufzubringenden Flächenstück aus einem nicht leitfähigen, flexiblen Material, in welchem - an einen Sensor angeschlossen - eine mit demselben verbundene Strom- bzw. Spannungsliefereinheit, eine Sensordatenmess- und -verarbeitungseinheit und eine mit einer Antenne verbundene Kommunikationseinheit eingebettet sind.

Die neue Vorrichtung ist also darauf gerichtet, einerseits die Feuchte der Haut und deren zeitliche Veränderung und andererseits den Status einer Hautläsion, Wunde nach Verletzung oder Operation, krankhaften Hautveränderung u. dgl. und deren zeitliche Veränderung durch Ermittlung bzw. Monitoring der Haut-Impedanz zu ermitteln.

Es ist schon lange bekannt, dass eine der wesentlichen Faktoren für Aussehen und Wohlbefinden einer Person im Gehalt von deren bekleidungsfreien Hautstellen, insbesondere von deren Gesichtshaut, an Feuchte begründet ist.

Auf diesem Wissen beruht ein Großteil der Forschung, der Altersforschung und insbesondere der Bemühungen des Kosmetiksektors, wobei die wesentliche Absicht darin besteht, die Nachhaltigkeit, insbesondere von jeweils neu entwickelten bzw. neu zu entwickelnden Hautlotionen, -emulsionen, -cremen und -salben, zu verbessern.

Für die Ermittlung der Effektivität und Wirkung von auf die Haut aufzubringenden Kosmetika und gegebenenfalls Arzneizubereitungen, kosmetischen Produkten und Rohstoffen hierfür sind die Bestimmung der Hautfeuchtigkeit, der Hautelastizität und des transepidermalen Wasserverlusts (TEWL) und deren/dessen Veränderungen innerhalb einer jeweils vorbestimmten Beobachtungsperiode weit verbreitete Methoden.

An sich sind heute für die Ermittlung der Hautfeuchtigkeit verschiedene Verfahren, wie insbesondere Infrarotspektroskopie, Resonanzfrequenzmessung, NMR-Methoden, Messung des elektrischen Widerstandes, der Impedanz oder der Kapazität der Haut üblich. Der Messfühler eines Kapazitätsmessgerätes für die Bestimmung der Hautfeuchte enthält zumindest einen Kondensator. Wird der Gerätekopf auf die Haut gedrückt, so gelangt deren Hornschicht in den Streubereich des Kondensatorfeldes, wobei man sich die relativ hohe Dielektrizitätskonstante von Wasser im Bereich von etwa 80 bei 30°C zunutze macht. Im Prinzip wird die Dielektrizitätskonstante der Haut bestimmt, die größer ist, wenn die Haut feuchter ist und umgekehrt. Der Wassergehalt der Haut ändert den Kapazitätswert. Derselbe wird im Gerät in einen digitalen Messwert umgewandelt, der dem jeweiligen Feuchtigkeitsgehalt der Haut entspricht.

So wurde in diesem Zusammenhang beispielsweise festgestellt, dass bei einer Coenzym Q10-haltigen Creme, welche einmal keinen und einmal einen nanostrukturierten Lipid Carrier (NLC) enthielt, sich die bleibenden Hautfeuchtewerte voneinander deutlich unterscheiden. Es wurde gefunden, dass die Hautfeuchtigkeit durch das Auftragen jeder der Cremes nach sieben Tagen deutlich ansteigt. Sie steigt bei Auftragen der NLChaltigen Creme über den gesamten Studienzeitraum an, während sie bei Anwendung der NLC-freien Creme zuerst ansteigt und dann auf einem konstanten niedrigen Wert verbleibt.

Die neue Vorrichtung ist aber keineswegs auf die Beobachtung der Wirkung von Kosmetika auf die Haut beschränkt, sondern ist insbesondere auch darauf ausgerichtet, Veränderungen der Haut, seien dieselben durch Wunden, Läsionen, Mikroorganismenbefall oder gegebenenfalls durch innersekretorische Veränderungen bedingt, durch Messung der Impedanz des Widerstandes und/oder der Kapazität der Haut zu ermitteln.

Aus der WO 2013/049716 A1 ist ganz allgemein eine flexible Vorrichtung mit einem Sensor für die Ermittlung und letztlich für die Anzeige der allgemein auf einer Oberfläche und in deren Nahbereich, insbesondere einer Hautfläche, herrschenden physikalischen und/oder chemischen Verhältnisse bzw. Bedingungen bekannt geworden, wobei dort an dem jeweiligen für seine Mess-Aufgabe vorgesehenen Sensor eine Verarbeitungseinheit für die von demselben gelieferten Daten und eine Kommunikationseinheit mit Antenne, insbesondere für die Weitergabe der berechneten Daten nach außen, also an ein übliches Kommunikationsgerät, wie Handy, Smartphone od. dgl., angeschlossen ist.

Die US 2011/178375 beschreibt eine Vorrichtung für die Ermittlung des Zustandes der Haut einer Person mit einer an zumindest einer Stelle der Haut auf dieselbe unter Bindung an dieselbe aufzubringenden, an dieselbe ortsfest flächig bindbaren selbstklebenden, Folien-Flächenstück aus einer hauflächentopographie-anpassenden, selbst nicht leitfähigen Kunststoff- und/oder polymergebundenen, Textilgewebefolie, in welcher - an einen Sensor angeschossen - eine mit demselben verbundene Strom- bzw.

Spannungsliefereinheit, eine Sensordatenmess- und -verarbeitungseinheit und eine mit einer Antenne verbundene Kommunikationseinheit eingebettet sind,
- mit einer auf demselben im Wesentlichen mittig angeordneten, das Folien-, insbesondere Hautpflaster-Flächenstück quer durchsetzenden Ausnehmung für eine visuelle Beobachtung einer jeweiligen Hautstelle und/oder für ein mehrmaliges Aufbringen einer kosmetischen und/oder medikamentösen Zubereitung von außen (as) direkt auf die Haut der Person ausgebildet ist,
- wobei als Sensor an dem Folien-Flächenstück hautseitig und direkt auf die Haut auflegbar, zumindest ein Paar von zumindest zwei einander zugeordneten, flächengleichen, voneinander beabstandeten Kontakt-Elektroden angeordnet sind,
- welche jeweils über innerhalb der Folie verlegte Leitungen mit einer dort angeordneten, von außen (as), insbesondere mittels NFC-Technik, mit Strom bzw. Spannung versorgbaren, Spannungsliefereinheit und mit zumindest einer mit derselben elektrisch verbundenen Impedanz-, Widerstands- oder Kapazitäts-Messeinheit verbunden sind,
- von welcher aus eine passive Kommunikationseinheit mit von derselben ausgehender, ebenfalls innerhalb der Folie des Flächenstücks, zumindest um die Ausnehmung herum geführter und entlang der nahe den Ränder(n) des Folien-Flächenstücks verlegter, Antennenschleife mit den Widerstands- und/oder Kapazitäts-Messdaten versorgbar ist,
- welche mittels NFC-fähigem Kommunikationsgerät, aus der Gruppe Handy, Smartphone, oder Reader von außen her direkt als Hautfeuchte- und/oder Hautläsions- oder - wunden-Status-Daten, und deren zeitliche Veränderung, abrufbar sind.

Die Erfindung hat sich nun die Aufgabe gestellt, eine praktisch handhabbare, nicht aufwendig herstellbare und zu bedienende, auf die zu beobachtende Hautstelle aufzubringende und dort einige Zeit und ohne Auswechslung verbleiben könnende Vorrichtung zur längerfristigen, auch visuellen Beobachtung der Reaktion bzw. des Verhaltens der Haut, beispielsweise nach deren kosmetischer Behandlung oder des Verhaltens einer Wunde nach deren, gegebenenfalls mehrmals wiederholter, medizinischer Behandlung zu schaffen, welche eine differenzierte, klare Information über den Zustand der Haut und über durchaus geringe Änderungen des Hautzustandes zu liefern im Stande ist.

Gegenstand der Erfindung ist eine wie soeben beschriebene, aus dem Stand der Technik bekannte, Vorrichtung zur Ermittlung des Hautzustands, welche sich von dieser bekannten Vorrichtung ganz wesentlich dadurch unterscheidet,
- dass im Folien-Flächenstück- zumindest zwei mal zwei bzw. zwei Paare von einander diametral gegenüberliegenden, jeweils einander zugeordneten, also insgesamt zumindest vier Elektroden, deren einander gegenüberliegende Ränder oder Kanten geradlinig sind und zueinander parallel verlaufen, jeweils im Nahbereich von je zwei einander gegenüber angeordneten Rändern der Ausnehmung angeordnet sind, wobei die Elektroden mit der Impedanz-, Widerstands- oder Kapazitätsmesseinheit verbunden sind.

Wenn, wie heute üblich, ein Kommunikations-Empfangsgerät, wie Handy, Reader oder Smartphone mit entsprechender Messdaten-Verarbeitungs-"App" für die Umrechung von Impedanz-, Widerstands- und/oder Kapazitätsmessdaten z.B. in Feuchte- oder andere charakteristische Hautparameterdaten zur Verfügung steht, kann die Unterbringung der Messdatenverarbeitungs- bzw. Recheneinheit im neuen für die Bestimmung der Hautfeuchte vorgesehenen Folien-, insbesondere Hautpflaster-Flächenstück, wo ohnedies äußerst wenig Platz für die Unterbringung von Hardware-Komponenten zur Verfügung steht, auch unterbleiben.

An dieser Stelle ist zur Erfindung im Lichte des Standes der Technik ergänzend folgendes auszuführen:
Der wesentliche Vorteil der erfindungsgemäßen Vorrichtung besteht insbesondere in der Form der das Folien-, insbesondere Hautpflaster-Flächenstück durchsetzenden Ausnehmung. Durch dieselbe ist es zum ersten Mal möglich, dass - ohne jeweils das gesamte Folien-, insbesondere Hautpflaster-Flächenstück immer wieder von der Haut ablösen zu müssen, und zwar, wenn beispielsweise ein zu testendes Kosmetikum in die Haut eingezogen und wieder eine neue Portion Kosmetikum aufzutragen ist - eine neue Portion Kosmetikum von außen direkt auf den innerhalb der Ausnehmung freiliegenden und somit von außen zugänglichen Haut- oder Hautwundenbereich aufgebracht werden kann und in der dadurch ermöglichten besonderen Art der Anordnung der zumindest 2 Paare von einander zugeordneten Elektroden.

Mit dem die Ausnehmung aufweisenden "Dauerpflaster" mit der neuartigen Anordnung der Elektrodenpaare ist es ermöglicht, den Fortgang, beispielsweise einer Wundheilung, gegebenenfalls nach periodisch wiederholter Aufbringung einer medizinischen Wundsalbe, eines Wundpulvers od. dgl. zu beobachten und zu kontrollieren, wobei trotz Verzichtes auf ein, wie bisher nötig gewesenes Wechseln des Pflasters jederzeit eine visuelle Kontrolle, z.B. des Heilungsverfahrens ermöglicht ist. Als Beispiele für derartige "Wunden" seien Brand-, Schnitt-, chronische Wunden, wie z.B. diabetischer Fuß, Dekubitus, Ulkus, Lupus od. dgl. genannt.

Damit ist - da ja das Folien-, insbesondere Hautpflaster-Flächenstück nicht mehr jedes Mal für einen Kosmetik- oder Wundheilungsmittelauftrag abgelöst werden muss und an dessen Stelle ein neues derartiges neu aufzubringen ist - voll gewährleistet , dass der neue Auftrag des Kosmetikums ganz exakt an jener Stelle bzw. in jenem Bereich der Haut erfolgen kann, wo schon vorher, meist schon mehrmals, ein Kosmetikum, eine pharmazeutische Zubereitung, also z.B. eine Creme oder Salbe, aufgebracht worden ist.

Weiters befinden sich die Messelektroden bei jeder der mit zeitlichen Abständen aufeinander folgenden Messungen immer exakt an ein- und derselben Stelle, wodurch eine hohe Konstanz im Mess-Setup erreicht werden kann. Dies ist hochrelevant, da bekannt ist, dass die Impedanz stark von der Distanz zwischen den Elektroden abhängig ist, ebenso die Eindringtiefe des Messstromes und damit das vermessene Gewebsvolumen. Beim manuellen Kleben von Klebeelektroden muss man sich deren Positionen genau merken oder besser, direkt auf dem jeweiligen Körperteil "einzeichnen", wobei die Gefahr der Variationen, wie z.B. infolge unterschiedlicher Positionen, unterschiedlicher Elektrodeneigenschaften, wie Typ, Alterung od. dgl., unterschiedlicher Anpressdruck etc. bei jedem Durchgang sehr groß ist.

Durch die, gemäß der vorliegenden Erfindung, Kennzeichen des neuen Anspruchs 1 vorgesehene, mehrachsig "symmetrische" Anordnung und Ausbildung mehrerer Elektroden mit geradlinigen, einander zugekehrten, zueinander parallelen Rändern wird der für den Dermatologen und dessen Anamnese vorteilhafte neue Effekt erzielt, dass im Vergleich zu bisher bekannten Pflastern ein größeres, sehr homogenes, parallel verlaufende und einander kreuzende Feldlinien aufweisendes elektrisches Feld zur Verfügung steht und dadurch eine Mittelung der einzelnen punktweise auftretenden Haut- bzw. Hautwundzustandswerte erreicht wird, wobei weiters der wesentliche Vorteil gegeben ist, dass jegliche zeitliche Veränderung in der Topografie des Zustandes der in Beobachtung stehenden Hautfläche registrierbar ist und darauf in jeweils geeigneter Weise reagiert werden kann.

Die erfindungsgemäß vorgesehene Anordnung und Ausbildung der Elektroden von Hautstatus-Ermittlungspflastern ist dem Stand der Technik absolut nicht zu entnehmen und ist dort auch nicht im Entferntesten angedacht.

Zu den weiteren Vorteilen der Erfindung sei Folgendes näher ausgeführt:
- Wesentliche Erleichterung der Anwendung beim Wound Assessment.
- Man muss das Pflaster nicht immer wieder ablösen, daher wird der Wundbereich nicht gereizt.
- Die Wunde kann wahlweise offen liegen bleiben (Lufttrocknung) oder auch abgedeckt werden, z.B. mittels, beispielweise transparenter, Folie, Textil od. dgl.
- Die Wunde kann durch die Ausnehmung jederzeit inspiziert werden und mit Cremes, Puder od. dgl. behandelt werden.
- Dennoch ist das Mess-Setup, im Vergleich zu einem, wie bisher üblichen, manuellen Kleben einzelner Klebeelektroden, automatisch konstant.

Was die Einsatzgebiete der neuen Vorrichtung betrifft, so kommen hierfür einerseits wissenschaftliche Forschung und Entwicklung, insbesondere auf den Gebieten Kosmetik und Medizin und andererseits zu einem wesentlich größeren Teil, Praxis-Tests an Personen und insbesondere auch deren Behandlung, also insbesondere im Rahmen des Wound Assessments in Frage.

Mit der Erfindung ist also ein wesentlich besser gesicherte Messergebnisse erbringender, besonders zu bevorzugender, Parallelverlauf der Feldlinien zwischen den Elektroden jedes Elektrodenpaares gewährleistet.

Gemäß einer vorteilhaften Ausführungsform der Erfindung ist vorgesehen, dass der Abstand zwischen den einander diametral gegenüberliegenden Rändern der Ausnehmung und den ausnehmungs-randnahen einander diametral gegenüberliegenden Rändern oder Kanten der Elektroden zwischen 2 und 10 mm beträgt und/oder das Verhältnis der Öffnungsweite der Ausnehmung zwischen den ausnehmungsnahen Rändern derselben und dem Abstand der einander zugekehrten, zueinander parallel verlaufenden Ränder der Kontakt-Elektroden mindestens 3 zu 10, beträgt, wobei die Länge der ausnehmungsrand-nahen Ränder der einzelnen Elektroden 75 bis 125% der Längenausdehnung der einzelnen elektrodennahen Seiten der geradlinige Ränder aufweisenden Ausnehmung oder der Sehnen der dem Kreis einer kreisrunden Ausnehmung eingeschriebenen Sehnen der - der Zahl der Elektroden entsprechenden - Kreissektoren beträgt.

Es soll an dieser Stelle betont werden, dass die oben genannten Formen der Ausnehmung wesentlich sind.
Günstige Anordnungen der Elektroden bzw. Elektrodenpaare können folgende sein:
- mehrere Paare (Zweileitermethode) oder Quadrupel (Vierleitermethode) parallel, zum Zwecke der Redundanz, oder um eine eindimensionale Diskretisierung zu ermitteln
- zwei oder mehrere Paare (Zweileitermethode) oder Quadrupel (Vierleitermethode) orthogonal zueinander (hilfreich zur Erkennung und Beurteilung von Anisotropien)
- mehrere, vorzugsweise 8 Paare oder Quadrupel oktagonal bzw. kreisförmig um eine kreisrunde Ausnehmung angeordnete Elektroden für die Erzeugung zweidimensionaler Maps mittels mathematischer Methoden (z.B. lineare Rückprojektion). Konkreter Anwendungsfall: z.B. Monitoring der Entwicklung von Wundgrößen.

Was die Stromversorgung der Elektroden betrifft, so kommen hierfür z.B. folgende Varianten bzw. Methoden in Frage: Gleichstrom und Wechselstrom, Konstantstrom- auch Konstantspannungsmethode, sowohl singlefrequent, multifrequent oder ,sweep' (typisch: 0 bis 2 MHz).

Weiters ist es von Vorteil, wenn zumindest eines der Paare von Elektroden mit je zwei Sub-Elektroden, jeweils eine für die Stromeinspeisung und eine für die Spannungsmessung, pro Elektrode ausgebildet ist.

Um Einflüsse und Störungen der Messergebnisse durch Außeneinflüsse möglichst zu vermeiden, ist günstiger Weise vorgesehen, dass innerhalb einer Schicht zwischen den Oberseiten der Elektroden, der Spannungsliefereinheit, der Messeinheit, der Kommunikationseinheit und deren leitenden Verbindungen miteinander einerseits und der, möglichst nahe dem Außenrand oder um die Ausnehmung im Raum zwischen deren Rand und dem ausnehmungsnahen Rand der Elektroden des Folien-Flächenstücks verlegten, ringartig in sich geschlossenen NFC-Antenne anderseits, eine flächige Abschirmungseinheit in Form eines feinen Netzes oder einer Folie aus einem elektrisch leitfähigen Material angeordnet ist.

Um reproduzierbare Ergebnisse zu erhalten, ist es vorteilhaft, für einen guten Flächenkontakt zwischen Mess-Elektroden und Haut der jeweils zu testenden Person zu sorgen, was dadurch erreichbar ist, dass die direkt auf die Haut aufzubringenden Unterseiten der Elektroden mit einer Kontaktverbesserungsschicht versehen oder versehbar sind.

Insbesondere aus Platzgründen ist es von Vorteil, wenn Impedanz-, Widerstands- oder Kapazitätsmesseinheit, eine gegebenenfalls vorhandene zentrale Messdatenverarbeitungseinheit, und die Kommunikationseinheit innerhalb eines gemeinsamen Chips angeordnet sind.

An sich kann die Antenne überhaupt neben der Messeinheit im Pflaster angeordnet sein, was zwar die Fläche des Pflasters vergrößert, aber praktisch nicht stört.

Weiters sei erwähnt, dass die Stromversorgung von deren Messeinheiten und den sonstigen Komponenten mittels Induktion bzw. magnetischer Kopplung durch das Kommunikationsgerät von außen oder gegebenenfalls mittels einer in das Folien-, insbesondere Hautpflaster-Flächenstück integrierten Minibatterie erfolgen kann.

Schließlich ist - insbesondere, um Irritationen der oft empfindlichen Haut durch die neue pflasterartige Vorrichtung möglichst zu vermeiden - vorgesehen, dass das Folien-, insbesondere Hautpflaster-Flächenstück, unter- bzw. hautseitig mit einem hautfreundlichen Selbstklebematerial beschichtet ist.

Anhand der Zeichnung wird die Erfindung näher erläutert.

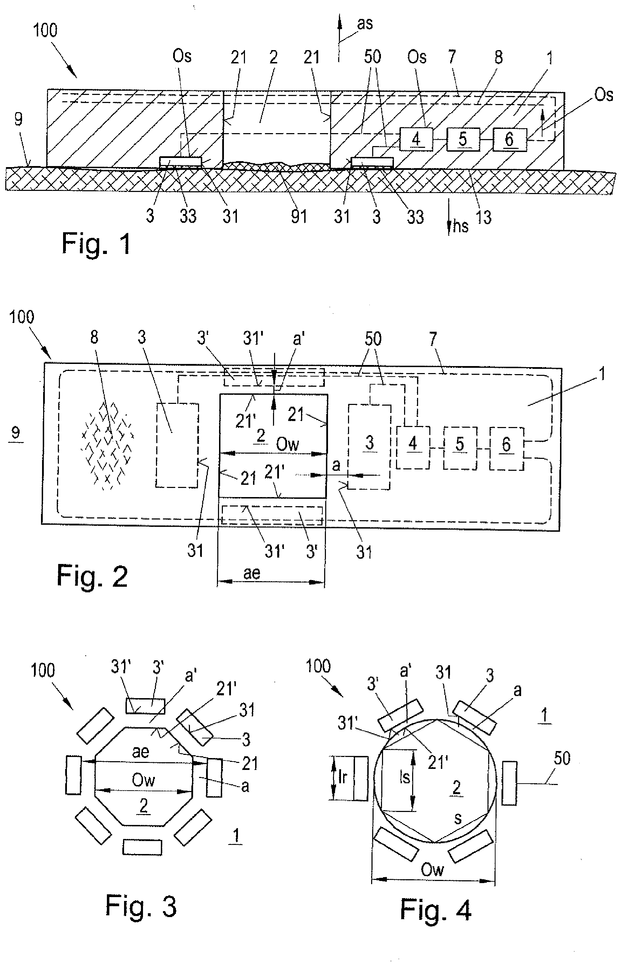

Die Fig. 1 und Fig. 2 zeigen in Schnittansicht und Draufsicht eine erfindungstypische pflasterartige Vorrichtung 100, beispielsweise zur Hautfeuchtebestimmung, deren Folien-, insbesondere Hautpflaster-Flächenstück 1 mittels einer Haft- bzw. (Selbst-)Klebeschicht 13 flächig auf einem z.B. für eine Untersuchung vorgesehenen Bereich der Haut 9 einer Person aufgebracht ist, die Fig. 3 bis 4 verschiedene weitere mögliche Formen von Elektrodenanordnungen und Ausnehmungen.

Gut aus den beiden Fig. 1 und Fig. 2 ist die, das mit einer nicht leitenden Plastik- oder Gewebefolie gebildete Folien-, insbesondere Hautpflaster-Flächenstück 1 in dessen mittleren Bereich voll durchsetzende, hier nahezu quadratische, Ausnehmung 2 mit ihren jeweils einander gegenüberliegenden, zueinander parallelen Rändern 21, 21', entlang derer die einander zugekehrt gegenüberliegenden, hier ebenfalls zueinander parallelen Ränder 31 und 31' der einander zugeordneten Messelektroden 3 und 3' verlaufen, zu erkennen, wobei diese Messelektroden unterseitig bzw. hautseitig hs mit einer Kontaktverbesserungsschicht 33 versehen sind, und so direkt oder über die Kontaktverbesserungsschicht 33 mit der Haut 9 in elektrischen Kontakt gelangen.

Auf den längere Zeit zu beobachtenden von außen as durch die Ausnehmung 2 zugänglichen Hautbereich am Grund der Ausnehmung 2 ist eine Schicht eines z.B. zu testenden, die Haut 9 mit Feuchte versorgenden Kosmetikums 91 aufgetragen.

In gleicher Weise kann die Ausnehmung 2 über einer Wunde der Haut 9 angeordnet werden, wobei auch hierbei auf die Wunde periodisch eine Arznei-Salbe, ein derartiges Puder od. dgl. auftragbar ist.

Über Leitungen 50 sind die Elektroden 3, 3' mit einer Impedanz-, Widerstands- oder Kapazitäts-Messeinheit 4, einer Messdatenverarbeitungs- und Recheneinheit 5 und einer Kommunikationseinheit 6 verbunden, an welche ihrerseits eine um die Ausnehmung 2 herum geführte Leiterschleife bzw. Antenne 7 für die (Funk-)Verbindung mit einem nicht gezeigten externen Empfangsgerät, wie Handy, Reader oder Smartphone angeschlossen ist, mittels welchem die akuten Hautfeuchte- bzw. -zustandswerte abrufbar sind, wobei hier zwischen den genannten Komponenten 3 bis 6 bzw. deren Oberseite Os und der Leiterschleife 7 ein Abschirmungsnetz 8 od. dgl. eingezogen sein kann.

Die Fig. 3 und 4 zeigen verschiedene Formen der Ausnehmung 2 der neuen pflasterartigen Vorrichtung 100 und erfindungsgemäß mögliche Anordnungen der im Folienmaterial eingebetteten, unterseitig freien Elektroden 3, 3' um die Ränder 21, 21' der Ausnehmung 2.

Es zeigt die Fig. 3 eine Oktogonal-Form aufweisende Ausnehmung 2, wobei dort im Wesentlichen parallel zu den hier gleich langen Oktogon-Seiten s die rechteckigen Elektroden 3, 3' angeordnet sind.

Die Elektroden 3, 3' haben hier eine Längen-Ausdehnung Ir ihrer ausnehmungsnahen Ränder bzw. Kanten 31, 31' von etwa 100% der Länge Is der einzelnen Oktogonseiten s.

Die Fig. 4 zeigt eine Ausnehmung 2 in Kreisform mit darin eingezeichnetem Sechseck mit Sechsecksektion-Sehnen s mit den im Abstand a, a' vom Rand 21, 21' oder Ausnehmung sechs Elektroden 3, 3'. Deren Ausdehnung Ir entspricht hier zu 75% der Länge I der einzelnen in den Kreis eingeschriebenen Sektoren-Sehnen s.

## Patentansprüche

1. Vorrichtung für die Ermittlung des Zustandes der Haut einer Person mit einem an zumindest einer Stelle der Haut auf dieselbe unter Bindung an dieselbe aufzubringenden, an dieselbe ortsfest flächig bindbares selbstklebendes, Folien-Flächenstück (1) aus einer hautflächentopographie-anpassenden, selbst nicht leitfähigen Kunststoff- und/oder polymergebundenen, Textilgewebefolie, in welchem - an einen Sensor angeschlossen - eine mit demselben verbundene Strom- bzw. Spannungsliefereinheit, eine Sensordatenmess- und -verarbeitungseinheit und eine mit einer Antenne verbundene Kommunikationseinheit eingebettet sind,
- mit einer auf demselben mittig angeordneten, das Folien-Flächenstück (1) quer durchsetzenden Ausnehmung (2) für visuelle Beobachtung einer jeweiligen Hautstelle und/oder für ein mehrmaliges, Aufbringen einer kosmetischen oder medikamentösen Zubereitung (91) von außen (as) direkt auf die Haut (9) der Person ausgebildet ist,
- wobei als Sensor an dem Folien-Flächenstück (1) hautseitig (hs) und direkt auf die Haut (9) auflegbar, zumindest ein Paar von zumindest zwei einander zugeordneten, flächengleichen, voneinander beabstandeten Kontakt-Elektroden (3;3') angeordnet sind,
- welche jeweils über innerhalb der Folie verlegte Leitungen (50) mit einer dort angeordneten, von außen (as) mittels NFC-Technik, mit Strom bzw. Spannung versorgbaren, Spannungsliefereinheit (4) und mit zumindest einer mit derselben elektrisch verbundenen Impedanz- Widerstands- oder Kapazitäts-Messeinheit (5) verbunden sind,
- von welcher aus eine passive Kommunikationseinheit (6) mit von derselben ausgehender, ebenfalls innerhalb der Folie des Flächenstücks (1), zumindest um die Ausnehmung (2) herum geführter und entlang der nahe den, Ränder(n) (11) des Folien-Flächenstücks (1) verlegter, Antennenschleife (7) mit den Widerstands- und/oder Kapazitäts-Messdaten versorgbar ist,
- welche mittels NFC-fähigem, Kommunikationsgerät, aus der Gruppe Handy, Smartphone, oder Reader von außen (as) her direkt als Hautfeuchte- und/oder Hautläsions- oder -wunden-Status-Daten, und deren zeitliche Veränderung, abrufbar sind,
- dass die Ausnehmung (2) im Folien-Flächenstück (1) Rechteck- oder Quadrat-, Kreis-, Sechseck- oder Oktogonal-Form aufweist **dadurch gekennzeichnet**
- **dass** im Folien-Flächenstück (1) - zumindest zwei mal zwei bzw. zwei Paare von einander diametral gegenüberliegenden jeweils einander zugeordneten, also insgesamt zumindest vier Elektroden (3; 3'), deren einander gegenüberliegende Ränder (31, 31') oder Kanten geradlinig sind und zueinander parallel verlaufen, jeweils im Nahbereich von je zwei einander gegenüber angeordneten Rändern (21; 21') der Ausnehmung (2) angeordnet sind, wobei die Elektroden mit der Impedanz-, Widerstands- oder Kapazitätsmesseinheit (5) verbunden sind.

2. Hautstatus-Ermittlungs-Vorrichtung nach Anspruche 1, **dadurch gekennzeichnet,**
**dass** der Abstand (a; a') zwischen den Rändern (21; 21') der Ausnehmung (2) und den ausnehmungs-randnahen Rändern (31; 31') der Elektroden (3; 3') zwischen 2 und 10 mm beträgt und/oder das Verhältnis der Öffnungsweite (ow) der Ausnehmung (2) zwischen den ausnehmungsnahen Rändern (21, 21') derselben und dem Abstand (ae) der einander zugekehrten, zueinander parallel verlaufenden Ränder (31; 31') der Kontakt-Elektroden (3,3') mindestens 3 zu 10, beträgt, und
- wobei die Länge der ausnehmungsrand-nahen Ränder (31, 31') der einzelnen Elektroden (3, 3') 75 bis 125% der Längenausdehnung der einzelnen elektrodennahen Seiten der geradlinige Ränder aufweisenden Ausnehmung (2) oder der Sehnen der dem Kreis einer kreisrunden Ausnehmung (2) eingeschriebenen Sehnen der der Zahl der Elektroden entsprechenden Kreissektoren beträgt.

3. Hautstatus-Ermittlungs-Vorrichtung nach Anspruche 1 oder 2, **dadurch gekennzeichnet,**
**dass** zumindest eines der Paare von Elektroden (3, 3') mit je zwei Sub-Elektroden, jeweils eine für die Stromeinspeisung und eine für die Spannungsmessung, pro Elektrode (3, 3') ausgebildet ist.

4. Hautstatus-Ermittlungs-Vorrichtung nach einem der Ansprüche 1 bis 3, **dadurch gekennzeichnet,**
**dass** zwischen den Oberseiten (Os) der Elektroden (3; 3'), der Spannungsliefereinheit (4), der Messeinheit (5), der Kommunikationseinheit (6) und deren leitenden Verbindungen (50) miteinander einerseits und der, möglichst, nahe dem Außenrand (11') oder um die Ausnehmung (2) im Raum zwischen deren Rand (21, 21') und dem ausnehmungsnahen Rand (31, 31') der Elektroden des Folien-Flächenstücks (1) verlegten, ringartig in sich geschlossenen NFC-Antenne (7) anderseits, eine flächige Abschirmungseinheit (8) in Form eines feinen Netzes oder einer Folie aus einem elektrisch leitfähigen Material, angeordnet ist.

5. Hautstatus-Ermittlungs-Vorrichtung nach einem der Ansprüche 1 bis 4, **dadurch gekennzeichnet,**
**dass** die direkt auf die Haut (9) aufzubringenden Unterseiten der Elektroden (3; 3') mit einer Kontaktverbesserungsschicht (33) versehen oder versehbar sind.

6. Hautstatus-Ermittlungs-Vorrichtung nach einem der Ansprüche 1 bis 5, **dadurch gekennzeichnet,**
**dass** die Impedanz-, Widerstands- und/oder Kapazitätsmesseinheit (5) und die Kommunikationseinheit (6) innerhalb eines gemeinsamen Chips angeordnet sind.

7. Hautstatus-Ermittlungs-Vorrichtung nach einem der Ansprüche 1 bis 6, **dadurch gekennzeichnet,**
**dass** die Stromversorgung von deren Messelektroden und den sonstigen Komponenten mittels elektromagnetischer Kopplung durch das Kommunikationsgerät von außen oder mittels einer in das Folien-Flächenstück (1) integrierten Minibatterie erfolgt.

8. Hautstatus-Ermittlungs-Vorrichtung nach einem der Ansprüche 1 bis 7, **dadurch gekennzeichnet,**
**dass** das Folien-, insbesondere Hautpflaster-Flächenstück (1) unter- bzw. hautseitig (hs) mit einem hautfreundlichen Selbstklebematerial (13) beschichtet ist.

## Claims

1. Device for determining the state of a person's skin, having a film piece (1) made of a non-conductive plastic material and/or polymer-bound textile fabric film which adapts to the topography of the skin surface, which is to be applied to at least one location on the skin and is bonded to this, being self-adhesively bondable fixed flat to the skin, in which are embedded, linked to a sensor: a current or voltage supply unit connected to the sensor, a sensor data measurement and processing unit and a communication unit connected to an antenna,
- having a recess (2) which is arranged centrally on the communication unit and passes transversely through the film piece (1), for visual observation of a respective skin location and/or for repeated application of a cosmetic or medicinal preparation (91) from the outside (as) directly onto the skin (9) of the person,
- wherein at least one pair of at least two contact electrodes (3; 3'), which are associated with one another, coextensive, and spaced apart from one another, are arranged as sensors on the film piece (1) on the skin side (hs) and can be laid directly on the skin (9),
- which electrodes are connected respectively via lines (50) laid inside the film to a voltage supply unit (4) arranged there, which can be supplied with current or voltage from the outside (as) using NFC technology, and to at least one impedance, resistance or capacitance measuring unit (5) connected electrically to the voltage supply unit,
- from which a passive communication unit (6) can be supplied with the resistance and/or capacitance measurement data by an antenna loop (7) originating from the communication unit, guided likewise within the film of the flat piece (1), at least around the recess (2), and laid along this proximal to the borders (11) of the film piece (1),
- which can be retrieved from outside (as) directly as data about skin moisture and/or skin lesion or wound status, and the changes in these over time, using an NFC-enabled communication device from the group containing mobile phone, smartphone or reader
- in that the recess (2) in the film piece (1) has a rectangular or square, circular, hexagonal or octagonal shape, **characterised**
- **in that** at least two times two or two pairs, i.e. a total of at least four, diametrically opposite and respectively associated electrodes (3; 3') are arranged in the film piece (1), whose mutually opposite borders (31, 31') or edges are rectilinear and run parallel to one another, and are respectively arranged in the vicinity of two mutually opposite borders (21; 21') of the recess (2), wherein the electrodes are connected to the impedance, resistance or capacitance measuring unit (5).

2. Skin state determination device according to claims 1, **characterised**
**in that** the distance (a; a') between the borders (21; 21') of the recess (2) and the recess-border-proximal borders (31; 31') of the electrodes (3; 3') is between 2 and 10 mm and/or the ratio of the opening width (ow) of the recess (2) between the recess-proximal borders (21, 21') of the recess (2) and the distance (ae) between the mutually facing and parallel-running borders (31; 31') of the contact electrodes (3, 3') is at least 3 to 10, and
- wherein the length of the recess-border-proximal borders (31, 31') of the individual electrodes (3 3') is 75 to 125% of the length of the individual electrode-proximal sides of the recess (2) having rectilinear borders, or of the chords of the chords inscribed on the circle of a circular recess (2) of the circular sectors corresponding to the number of electrodes.

3. Skin state determination device according to claims 1 or 2, **characterised**
**in that** at least one of the pairs of electrodes (3, 3') is designed with two sub-electrodes respectively for each electrode (3, 3'), one for the current feed and one for the voltage measurement.

4. Skin state determination device according to any one of claims 1 to 3, **characterised**
**in that** between the top sides (Os) of the electrodes (3; 3'), the voltage supply unit (4), the measuring unit (5), the communication unit (6) and their conductive connections (50) to one another on the one hand, and on the other hand the annular, self-contained NFC antenna (7), if possible laid close to the outer border (11') or around the recess (2) in the space between its border (21, 21') and the recess-proximal border (31, 31') of the electrodes of the film piece (1), a flat shielding unit (8) is arranged, in the form of a fine mesh or a film made of an electrically conductive material.

5. Skin state determination device according to any one of claims 1 to 4, **characterised**
**in that** the undersides of the electrodes (3; 3'), which are to be applied directly to the skin (9), are provided or can be provided with a contact improving layer (33).

6. Skin state determination device according to any one of claims 1 to 5, **characterised**
**in that** the impedance, resistance and/or capacitance measuring unit (5) and the communication unit (6) are arranged within a common chip.

7. Skin state determination device according to any one of claims 1 to 6, **characterised**
**in that** the power supply of the measuring electrodes and the other components takes place by means of electromagnetic coupling by the communication device from the outside or by means of a mini battery integrated in the film piece (1).

8. Skin state determination device according to any one of claims 1 to 7, **characterised**
**in that** the film piece (1), in particular the dermal patch piece, is coated on the underside or skin side (hs) with a skin-friendly self-adhesive material (13).

## Revendications

1. Dispositif de détermination de l'état de la peau d'une personne, avec une pièce plane du genre feuille (1) qui doit être appliquée au niveau d'au moins un endroit de la peau sur celle-ci tout en étant liée à celle-ci, qui est autoadhésive et peut être liée par sa surface au même endroit à ladite peau, qui est constituée d'une feuille adaptée à la topographie de la surface de la peau, en une matière plastique elle-même non conductrice et/ou en un tissu textile à liaison polymère, et dans laquelle sont intégrées - raccordées à un capteur - une unité fournissant courant ou tension et reliée à celui-ci, une unité de mesure et de traitement de données de capteur et une unité de communication reliée à une antenne,
- avec un évidement (2), disposé sur ladite pièce plane du genre feuille de manière centrée et traversant transversalement la pièce plane du genre feuille (1), destiné à l'observation visuelle d'un endroit de peau respectif et/ou à une application réitérée d'une préparation cosmétique ou médicamenteuse (91), depuis l'extérieur (as), directement sur la peau (9) de la personne,
- dans lequel est agencée comme capteur au niveau de la pièce plane du genre feuille (1), côté peau (hs) et pouvant être posée directement sur la peau (9), au moins une paire d'au moins deux électrodes de contact (3 ; 3') associées l'une à l'autre, de surface identique et distantes l'une de l'autre,
- lesquelles électrodes sont reliées, à chaque fois par l'intermédiaire de conducteurs (50) posés à l'intérieur de la feuille, à une unité fournisseuse de tension (4) agencée là et pouvant être alimentée de l'extérieur (as) en courant ou tension au moyen d'une technique NFC et à au moins une unité de mesure d'impédance, de résistance ou de capacité (5) reliée électriquement à celle-ci,
- à partir de laquelle peut être alimentée en données de mesure de résistance et/ou capacité une unité de communication passive (6) avec une boucle d'antenne (7) sortant de celle-ci, guidée également à l'intérieur de la feuille de la pièce plane (1) au moins autour de l'évidement (2) et posée le long près des bords (11) de la pièce plane du genre feuille (1),
- lesquelles données ainsi que leur variation dans le temps peuvent être interrogées directement de l'extérieur (as) au moyen d'un appareil de communication compatible NFC, du groupe des téléphones portables, smartphones ou lecteurs, en tant que données d'humidité de la peau et/ou d'état de lésion ou blessure de la peau,
- dans lequel l'évidement (2) dans la pièce plane du genre feuille (1) a une forme rectangulaire ou carrée, ronde, hexagonale ou octogonale,
**caractérisé en ce que**
- dans la pièce plane du genre feuille (1), au moins deux fois deux électrodes ou deux paires d'électrodes, donc au total au moins quatre électrodes (3 ; 3'), associées à chaque fois l'une à l'autre et disposées de manière diamétralement opposée l'une à l'autre, dont les bords ou arêtes (31, 31') en face les uns des autres sont droits et s'étendent parallèlement les uns aux autres, sont agencées à chaque fois à proximité de deux bords (21 ; 21'), agencés en face l'un de l'autre, de l'évidement (2), lesquelles électrodes sont reliées à l'unité de mesure d'impédance, de résistance ou de capacité (5).

2. Dispositif de détermination de l'état de la peau selon la revendication 1, **caractérisé en ce que**
- la distance (a ; a') entre les bords (21 ; 21') de l'évidement (2) et les bords (31 ; 31'), proches des bords de l'évidement, des électrodes (3 ; 3') est comprise entre 2 et 10 mm et/ou le rapport de la largeur d'ouverture (ow) de l'évidement (2) entre les bords (21, 21'), proches de l'évidement, dudit évidement et la distance (ae) des bords (31; 31'), en regard l'un de l'autre et parallèles l'un à l'autre, des électrodes de contact (3, 3') est égal à un rapport d'au moins 3 à 10, et
- la longueur des bords (31, 31'), proches des bords de l'évidement, des différentes électrodes (3, 3') est égale à 75 à 125 % de la dimension longitudinale des différents côtés, proches des électrodes, de l'évidement (2) présentant des bords droits ou des cordes, inscrites dans le cercle d'un évidement (2) rond, des secteurs de cercle correspondant au nombre des électrodes.

3. Dispositif de détermination de l'état de la peau selon la revendication 1 ou 2, **caractérisé en ce qu'**au moins l'une des paires d'électrodes (3, 3') est réalisée avec à chaque fois deux sous-électrodes, respectivement une pour l'alimentation en courant et une pour la mesure de tension, par électrode (3, 3').

4. Dispositif de détermination de l'état de la peau selon l'une quelconque des revendications 1 à 3, **caractérisé en ce qu'**une unité de blindage (8) plane sous la forme d'un fin treillis ou d'une feuille en un matériau électriquement conducteur est agencée entre d'une part les faces supérieures (Os) des électrodes (3 ; 3'), de l'unité fournisseuse de tension (4), de l'unité de mesure (5), de l'unité de communication (6) et de leurs liaisons conductrices (50) mutuelles et d'autre part l'antenne NFC (7) qui est posée le plus près possible du bord extérieur (11') ou autour de l'évidement (2) dans l'espace entre son bord (21, 21') et le bord (31, 31'), proche de l'évidement, des électrodes de la pièce plane du genre feuille (1) et qui est fermée sur elle-même à la manière d'un anneau.

5. Dispositif de détermination de l'état de la peau selon l'une quelconque des revendications 1 à 4, **caractérisé en ce que** les faces inférieures, devant être appliquées directement sur la peau (9), des électrodes (3 ; 3') sont munies ou peuvent être munies d'une couche (33) améliorant le contact.

6. Dispositif de détermination de l'état de la peau selon l'une quelconque des revendications 1 à 5, **caractérisé en ce que** l'unité de mesure d'impédance, de résistance et/ou de capacité (5) et l'unité de communication (6) sont agencées à l'intérieur d'une puce commune.

7. Dispositif de détermination de l'état de la peau selon l'une quelconque des revendications 1 à 6, **caractérisé en ce que** l'alimentation en courant de ses électrodes de mesure et des autres composants s'effectue au moyen d'un couplage électromagnétique via l'appareil de communication depuis l'extérieur ou au moyen d'une batterie miniature intégrée dans la pièce plane du genre feuille (1).

8. Dispositif de détermination de l'état de la peau selon l'une quelconque des revendications 1 à 7, **caractérisé en ce que** la pièce plane du genre feuille (1), en particulier du genre pansement, est revêtue au-dessous c'est-à-dire côté peau (hs) avec un matériau autoadhésif (13) hypoallergénique.
